# EUROPEAN PATENT APPLICATION

(11) **EP 1 116 490 A1**
(43) Date of publication of application: **18.07.2001**
(21) Application number: 99931550.0
(22) Date of filing: 27.07.1999
(51) Int. Cl.: A61K 38/40

(54) **LIVER FUNCTION AMELIORATING AGENTS**

(30) Priority: 30.07.1998 JP 22943498
(71) Applicant: MORINAGA MILK INDUSTRY CO., LTD., Minato-ku, Tokyo-to 108-0014 (JP)
(72) Inventor: TOMITA, Mamoru, Morinaga Milk Industry Co., Ltd., Zama-shi, Kanagawa 228-0004 (JP); SHIMAMURA, Seiichi, Morinaga Milk Ind. Co., Ltd., Zama-shi, Kanagawa 228-0004 (JP); TOIDA, Tomohiro, Morinaga Milk Industry Co., Ltd., Zama-shi, Kanagawa 228-0004 (JP)
(74) Representative: Pett, Christopher Phineas
(86) International application number: JP9904005
(87) International publication number: WO0006192

(57) **Abstract**

The present invention provides an improving agent for hepatic functions containing a lactoferrin and/or a hydrolysate of lactoferrin and having no side effects.

## Description

### Technical Field

The present invention relates to an improving agent for hepatic functions containing lactoferrin as effective ingredient. More particularly, it relates to an improving agent for hepatic functions containing metal-unsaturated lactoferrin, metal-saturated lactoferrin or apolactoferrin (hereinafter, they may be sometimes referred to as "lactoferrins") and/or hydrolysate of lactoferrins as effective ingredients.

### Background Art

Hepatic functions lower by various causes and, in some cases, that results in death. Test of the hepatic functions is usually carried out by the measurement of glutamate oxaloacetate transaminase (hereinafter, abbreviated as GOT), glutamate pyruvate transaminase (hereinafter, abbreviated as GPT), alkaline phosphatase (hereinafter, abbreviated as ALP), total bilirubin, albumin and total cholesterol in plasma. Until now, with regard to therapeutic agents for lowering the hepatic functions, only Shosaikoto (a land of traditional herbal medicines for hepatic diseases comprising Bulpleurum falcatum and six more herbs), Stronger Neo-Minophagen C (trade mark; hereinafter, abbreviated as SNMC), ursodeoxycholic acid, etc. have been used. All of those therapeutic agents have low effectiveness and have adverse reactions such as diarrhea, nausea and emesis whereby their administration for a long term is difficult.

Recently, as therapeutic agents for hepatic diseases, there have been known a highly branched chain amino acid preparation [Akiharu Watanabe and Misako Okita, "Kanzobyo to Chiryo Eiyo" (Hepatic Diseases and Therapeutic Nutrition), Daiichi Shuppan, 1992], galactoligosaccharide and food fiber (Japanese Patent Provisional Publication No.151854/1991), lactulose and lactitol (Nippon Rinsho, volume 52, no. 1, pages 100-118, 1994; Rinsho Iyaku, volume 11, no. 7, pages 1439-1473, 1995), raffinose (Japanese Patent Provisional Publication No. 179087/1998), antibody to stress proteins (Japanese Patent No. 2,775,576), etc.

On the other hand, lactoferrin is a nontoxic and natural iron-binding protein (being able to bind to two iron ions per molecule) contained in tear, saliva, peripheral blood, milk, etc. and its molecular weight is 86,000 for bovine lactoferrin and 88,000 for human lactoferrin ["Seikagaku Jiten" (Dictionary of Biochemistry), Kazutomo Imabori and Tamio Yamakawa edt., Second Edition, page 1390, Tokyo Kagaku Dojin, 1990].

It has been known that lactoferrin has an antibacterial action to harmful microorganisms such as Escherichia coli, genus Candida and genus Clostridium (Journal of Pediatrics, volume 94, page 1, 1997). In addition, lactoferrin has been known to be effective in fixation of useful microorganisms such as genus Bifidus and genus Lactobacillus in intestines of human being and animals (Japanese Patent No. 2,532,911) and to be a growth factor for microorganisms of genus Bifidus (Japanese Patent Provisional Publication No. 225419/1990). Moreover, there have been disclosures for many uses of lactoferrin as given below.
1) Antibacterial agent, antiviral agent and antiparasitic agent:
   Japanese Patent Provisional Publication Nos. 83131/1986; 249931/1987; 56273/1988; 284133/1988; 44273/1989; 221319/1989; 233226/1989; 48534/1990; 191205/1990; 233619/1990; 181421/1991; 193708/1991; 220130/1991; 48956/1994; 199698/1994; 256211/1994; 316529/1994; 206701/1995; 316069/1995;2970/1997;40578/1997;165342/1997; and 59865/1998.
2) Hematopoietic agent:
   Japanese Patent Provisional Publication Nos. 125398/1987; 22525/1988; 6408/1990; 130060/1991; 8269/1992; 141067/1992; and 95100/1992.
3) Agent for skin and for external application:
   Japanese Patent Provisional Publication Nos. 135726/1989; 196529/1995; 59450/1996; 231462/1996; and 333260/1996.
4) Agent for mouth and teeth:
   Japanese Patent Provisional Publication Nos. 279266/1993; 506098/1996; and 217693/1996.
5) Ophthalmologic agent:
   WO 92/08477; and Japanese Patent Provisional Publication Nos. 301785/1996 and 30966/1997.
6) Immunopotentiator and immunostimulator:
   Japanese Patent Provisional Publication Nos. 178759/1993; 32743/1994; 145068/1994; and 507763/1995.

Examples of other known uses are anti-tumor agent (Japanese Patent Provisional Publication No. 51337/1988), growth promoter for gastric and intestinal tracts (Japanese Patent Provisional Publication No. 93534/1989), neutralizing agent for bacterial toxins (Japanese Patent Provisional Publication Nos. 207089/1990 and 501416/1993), age retardant (Japanese Patent Provisional Publication Nos. 58871/1992 and 124980/1993), blood pressure depressant (Japanese Patent Provisional Publication No. 316598/1992), anti-rheumatic agent (Japanese Patent Provisional Publication No. 186368/1993), promoter for nerve growth factor production (Japanese Patent Provisional Publication No. 32557/1993), potentiator for digestive tract cells (Japanese Patent Provisional Publication No.48955/1994), brain protector (Japanese Patent Provisional Publication No. 172200/1994), arteriosclerosis preventer (Japanese Patent Provisional Publication No. 234655/1994), stimulator for mutin production (Japanese Patent Provisional Publication No. 12473/1997), therapeutic agent for neovascular diseases (Japanese Patent Provisional Publication No. 194388/1997), agent for abnormal crypt (Japanese Patent Provisional Publication No. 25249/1998), oral suppressor for cancer metastasis (Japanese Patent Provisional Publication No. 59864/1998), therapeutic agent for endotoxin-induced diseases (Japanese Patent Provisional Publication No. 114675/1998) and preventive and/or therapeutic agent for gastrointestinal troubles (Japanese Patent Provisional Publication No.130164/1998).

However, it has not been known that lactoferrin is effective for improving the hepatic functions and no references therefore have been available as well.

### Disclosure of Invention

An object of the present invention is to provide an agent for improving the hepatic functions.

The present invention relates to an improving agent for hepatic functions containing lactoferrin and/or hydrolysate of lactoferrin as an effective ingredient.

In the improving agent for hepatic functions according to the present invention, it is one preferred embodiment that lactoferrin is selected from a group consisting of metal-unsaturated lactoferrin, metal-saturated lactoferrin and apolactoferrin or is a mixture of two or more thereof.

Thus, the present inventors have carried out an intensive study for the novel use of lactoferrin which has not been known yet and, during its course, it has been found that administration of lactoferrin is able to improve the lowered hepatic functions. With regard to this fact, the present inventors have carried out the follow-up studies repeatedly by means of animal experiments, scientifically certified that lactoferrin is effective for improvement of hepatic functions and accomplished the present invention.

The improving agent for hepatic functions according to the present invention shows a significant effect for improvement of hepatic functions and, since lactoferrin which is an effective ingredient thereof is contained in cow's milk, etc. and is a protein or a hydrolysate thereof which is daily taken, there is almost no adverse action even when it is taken continuously for a long term. In addition, when the improving agent for hepatic functions according to the present invention is used together with other therapeutic agents for hepatic diseases (such as Interferon and SNMC), it is possible to reduce the dose of such agents.

### Best Mode For Carrying Out the Invention

The lactoferrins used as an effective ingredient for the present invention may be that which is commercially available or may be lactoferrin which is separated by a conventional method such as an ion-exchange chromatography from colostrum, transitional milk, matured milk, late milk, etc. of mammals (such as human being, cattle, water buffalo, horse, goat and sheep) and defatted milk and whey, etc. which are processed products thereof, apolactoferrin prepared by removal of iron from lactoferrin by a conventional method and a metal-unsaturated lactoferrin or a metal-saturated lactoferrin which is prepared a partial or a complete chelation of apolactoferrin with metal such as iron, copper, zinc and manganese.

Natural human lactoferrin is unable to be produced in large quantities but human lactoferrin which is produced by recombinant fungus, recombinant milking cow (transgenic cow), etc. obtained by means of a recombinant DNA technique can be used in the present invention as well.

Hydrolysate of lactoferrins used as an effective ingredient of the present invention is manufactured by hydrolysis of the above lactoferrins with an acid or an enzyme. To be more specific, that will be as follows.

Hydrolysis with an acid is that one of lactoferrins is dissolved in water, pure water or the like in a concentration of 0.1-20% (by weight; hereinafter, % has the same meaning unless otherwise mentioned) or, preferably, 5-15%, an acid (inorganic acid such as hydrochloric acid and phosphoric acid or organic acid such as citric acid) is added to the resulting solution to adjust the pH of the solution to 1-4 and the mixture is kept at a predetermined temperature to hydrolyze for a predetermined period. To be still more specific, when pH is adjusted to 1 to lower than 2, the mixture is kept at 80-130°C or, preferably, 90-120°C for 1-120 minute(s) or, when pH is adjusted to 2 to lower than 4, it is kept at 80-130°C or, preferably, 100-120°C for 1-120 minute(s) to conduct the hydrolysis. After that, the reaction solution is cooled and, if necessary, it may be neutralized, desalted and decolorized.

Hydrolysis using an enzyme is carried out in such a manner that one of the lactoferrins is dissolved in water, sterilized water, pure water, or the like in a concentration of 0.5-20% or, preferably, 5-15%, the pH is adjusted to that which is optimum to the enzyme used for the hydrolysis, an enzyme is added and the mixture is kept at the temperature which is optimum for the action of the enzyme to carry out the hydrolysis. There is no particular limitation for the enzyme used and commercially available endopeptidases such as Morusin (trade mark; manufactured by Seishin optimum pH: 2.5-3.0), swine pepsin (manufactured by Wako Pure Chemical; optimum pH: 2-3), Sumizyme (trade mark; manufactured by Shin Nippon Kagaku; optimum pH: 3.0), Amano M (trade mark; manufactured by Amano Seiyaku; optimum pH: 7.0) and trypsin (manufactured by Novo; optimum pH: 8.0) may be used either solely or jointly.

It is also possible to use the above enzyme together with commercially available soy sauce enzyme (manufactured by Tanabe Seiyaku) containing peptidase or exopeptidase derived from lactic acid bacteria manufactured by a method mentioned in Japanese Patent Publication No. 43878/1973 for example. Amount of the enzyme used to the substrate is within a range of 0.1-5.0% or, particularly preferably, 0.5-3.0%.

Hydrolysis using an enzyme is carried out by keeping the temperature at 15-55°C or, preferably, 30-55°C for 30-600 minutes or, preferably, 60-300 minutes. After that, the reaction solution as it is or after being neutralized is heated by a common method to inactivate the enzyme and, if necessary, decolorized to give a hydrolysate.

The improving agent for hepatic functions according to the present invention containing the above-mentioned lactoferrins and/or hydrolysate thereof as effective ingredient(s) can be made into preparations of various forms by known methods. Examples of the specific forms are tablets (including sugar-coated tablets, coated tablets and buccal tablets), diluted powder, capsules (including soft capsules), granules (including coated ones), pills, troches, liquid preparations and pharmaceutically acceptable sustained released preparations thereof.

The above preparations may be made into a pharmaceutical composition together with pharmacologically acceptable carrier, excipient, disintegrator, lubricant, coloring agent, etc. according to known methods for the manufacture of the pharmaceutical preparations. Examples of the carrier and the excipient which can be used for the preparations are lactose, glucose, refined sugar, mannitol, potato starch, corn starch, calcium carbonate, calcium phosphate, calcium sulfate, crystalline cellulose, licorice powder and gentian powder. Examples of the binder are starch, gelatin, syrup, polyvinyl alcohol, polyvinyl ether, polyvinylpyrrolidone, hydroxypropyl cellulose, ethyl cellulose, methyl cellulose and carboxymethyl cellulose.

Examples of the disintegrator are starch, agar, gelatin powder, carboxymethyl cellulose sodium, carboxymethyl cellulose calcium, crystalline cellulose, calcium carbonate, sodium bicarbonate and sodium alginate.

Examples of the lubricant are magnesium stearate, hydrogenated vegetable oil and Macrogol. Examples of the coloring agent are Red No.2, Yellow No.4 and Blue No.1 which are permitted to add to pharmaceuticals as coloring agents.

If necessary, tablets and granules may be coated with refined sugar, hydroxypropyl cellulose, pure shellac, gelatin, sorbitol, glycerol, ethyl cellulose, hydroxypropyl cellulose, hydroxypropyl methyl cellulose, polyvinylpyrrolidone, cellulose acetate phthalate, hydroxypropyl methyl cellulose phthalate, methyl methacrylate, methacrylic acid polymer, etc.

The improving agent for hepatic functions according to the present invention preferably contains the effective ingredient which is lactoferrin or decomposed product thereof in an amount of at least 1 mg per gram of the pharmaceutical preparation. Although the dose may vary depending upon age, symptom, etc., it is administered per os in an amount of at least 1 mg per kg of body weight of human being and administration of from 20 mg to 15 g per day is preferred.

The improving agent for hepatic functions according to the present invention is a component derived from milk and is highly safe and, therefore, when it is used together with other therapeutic agent for hepatic diseases such as Interferon and SNMC, dose of such other agent can be reduced and, in addition, a synergistic effect for improvement of hepatic functions can be expected.

The present invention will now be illustrated in detail by the following Test Examples.

### Test Example 1

This test was carried out for testing the improving effect of lactoferrin and hydrolysate thereof for hepatic functions.

### 1. Animals and Samples

### 1.1 Animals

Thirty male rats of Wistar strain (body weight: about 200 g) (purchased from Nippon Charles River) were randomly classified into five groups where each group consisted of six rats. They were subjected to a preliminary breeding for one week giving a solid feed (manufactured by Funabashi Nojo) and water to them freely.

### 1.2 Preparation Samples

With regard to lactoferrin, a commercially available product (manufactured by Morinaga Milk Industry) was used while, with regard to a hydrolysate of lactoferrin, that which was manufactured by the same method as in Referential Example was used.

### 2. Procedures

### 2.1 Control Group

One group which was randomly selected from the above-mentioned five groups was used and rats suffering from chronic liver injury were prepared as follows according to a method by Tamada, et al. (Kanzo, volume 28, no. 7, page 39, 1987). Thus, 50% carbon tetrachloride (manufactured by Wako Pure Chemicals) dissolved in olive oil (Japanese Pharmacopoeia) was subcutaneously administered to rats in an amount of 1.0 ml (0.5 ml as carbon tetrachloride) per kg of body weight twice a week for consecutive nine weeks.

### 2.2 Test Group

The rats of other four groups were treated as same as in the control group and, from the sixth week after initiation of administration of carbon tetrachloride, lactoferrin or a hydrolysate thereof dissolved in pure water was orally administered for consecutive three weeks in the dose as shown in Table 1 (500 mg/kg/day or 250 mg/kg/day).

### 2.3 Test of Hepatic Functions

After four days from the final administration of the sample, blood was collected from abdominal aorta under anesthetization with ether, then GOT, GPT and ALP in serum were measured using an automatic chemical analyzer (TBA-380 manufactured by Toshiba Medical) and average values for each group were calculated to carry out the test.

### 3. Results

Effect of lactoferrin to the models suffering from chronic liver injury induced by carbon tetrachloride is as shown in Table 1. The group administered with lactoferrin showed lower GOT, GPT and ALP as compared with the control group whereupon a high improving effect for hepatic functions was noted. In the case of a hydrolysate of lactoferrin, although the effect was a bit lower than lactoferrin, an apparent improving effect for hepatic functions was noted.

Tests were also carried out by changing the type of lactoferrins and hydrolysate thereof and nearly the same result was obtained.

**Table 1**

| Test Group | Dose (mg/kg/day) | GOT (IU/1) | GPT (IU/1) | ALP (IU/1) |
|---|---|---|---|---|
| Control Group | 0 | 635 | 223 | 883 |
| LF-Given Group | 250 | 268 | 83 | 525 |
| | 500 | 132 | 56 | 421 |
| Hydrolyzed LF-Given Group | 250 | 387 | 106 | 658 |
| | 500 | 211 | 64 | 535 |
| (Note) LF means lactoferrin | | | | |

### Test Example 2

This test was carried out for testing the improving effect of lactoferrin and hydrolysate thereof for acute hepatic disorder.

### 1. Animals and Samples

The same animals and samples as in Test Example 1 here used.

### 2. Procedures

Rats were tested as same as Test Example 1 method except the following procedures. 20% carbon tetrachloride (manufactured by Wako Pure Chemicals) dissolved in olive oil (Japanese Pharmacopoeia) was administered to rats into their abdominal cavities in an amount of 1.5 ml (0.3 ml as carbon tetrachloride) per kg of body weight one time. Lactoferrin or a hydrolysate thereof dissolved in pure water was orally administered for consecutive 5 days in the dose from the next day as shown in Table 2 (600 mg/kg/day or 350 mg/kg/day).

### 3. Results

Effect of lactoferrin and hydrolysate to the models suffering from acute liver injury induced by carbon tetrachloride is as shown in Table 2. The group administered with lactoferrin showed lower GOT, GPT and ALP as compared with the control group whereupon a high improving effect for hepatic functions was noted. In the case of a hydrolysate of lactoferrin, the same improving effect for hepatic functions was noted.

Tests were also carried out by changing the type of lactoferrin and a hydrolysate thereof and nearly the same result was obtained.

**Table 2**

| Test Group | Dose (mg/kg/day) | GOT (IU/1) | GPT (IU/1) | ALP (IU/1) |
|---|---|---|---|---|
| Control Group | 0 | 6365 | 1087 | 620 |
| LF-Given Group | 350 | 3235 | 556 | 387 |
| | 600 | 2162 | 212 | 265 |
| Hydrolyzed LF-Given Group | 350 | 3184 | 601 | 318 |
| | 600 | 2492 | 199 | 253 |
| (Note) LF means lactoferrin | | | | |

### Referential Example 1.

Human lactoferrin was manufactured from human milk by the following method mentioned in the published gazette (Japanese Patent Publication No. 13560/1994) of the Japanese Patent No. 2,130,919.

Thus, CM-Sephadex C-50 (manufactured by Pharmacia) (10 g) was swollen by water, the resulting ion exchanger of a sodium type (200 ml) was mixed with 10 liters of defatted human milk (pH: 6.7) and the mixture was stirred at 4°C for 16 hours and filtered to take out the ion exchanger. The ion exchanger taken out as such was washed with 2 liters of a 1.6% sodium chloride solution to remove impurities, then 1 liter of a 5.0% sodium chloride solution was added so that the component adsorbed with the ion exchanger was eluted to give 1.1 liters of a recovered liquid.

The resulting recovered liquid was dialyzed, sodium chloride was removed therefrom and the residue was freeze-dried to give about 12 g of human lactoferrin powder. Composition of the resulting human lactoferrin was 2.8% of water, 96.3% of lactoferrin and 0.5% of ash.

### Referential Example 2.

Commercially available bovine lactoferrin (manufactured by Morinaga Milk Industry) (500 g) was dissolved in 9 liters of pure water and adjusted to pH 2.5 with 0.1N hydrochloric acid, 10 g of commercially available porcine pepsin (manufactured by Sigma) were added and hydrolysis was carried out at 37°C for 6 hours. After that, pH was adjusted to 7.0 with 0.1N sodium hydroxide, the solution was heated at 75°C for 10 minutes to inactive the enzyme and centrifuged at 15,000 rpm for 30 minutes and the resulting supernatant liquid was freeze-dried by a common method to give about 420 g of hydrolyzed bovine lactoferrin.

Now, the present invention will be illustrated in more detail by way of the following Examples although the present invention is not limited thereto.

### Example 1: Preparation of Tablets Compounded with Bovine Lactoferrin

An improving agent for hepatic functions in a form of tablets having the following composition was manufactured by the following method.

| | |
|---|---|
| Bovine lactoferrin (by Morinaga Milk Industry) | 18.0 (%) |
| Lactose (by Morinaga Milk Industry) | 18.5 |
| Corn starch (by Nisshin Flour Milling) | 52.7 |
| Magnesium stearate (by Taihei Kagaku Sangyo) | 1.4 |
| Carboxymethyl cellulose calcium (by Gotoku Yakuhin) | 9.4 |

A mixture of bovine lactoferrin, lactose, corn starch and carboxymethyl cellulose calcium was uniformly kneaded together with addition of sterilized water appropriately and dried at 50°C for 3 hours, then magnesium stearate was added to the above-prepared dry product and mixed therewith and the mixture was tabletted by a common method to give tablets.

### Example 2: Preparation of Syrup Compounded with Bovine Lactoferrin

| | |
|---|---|
| Bovine lactoferrin (by Milei) | 8.0 (%) |
| Fructose/glucose liquid (by Sanmatsu Kogyo) | 12.4 |
| Citric acid (by Ueno Fine Chemicals) | 0.2 |
| Sodium citrate (by Maruzen Seiyaku) | 0.2 |
| Carboxymethyl cellulose calcium (by Gotoku Yakuhin) | 0.2 |
| Pure water (by Otsuka Pharmaceutical) | 79.0 |

The components were mixed and made into syrup by a common method.

### Example 3: Preparation of Bovine Lactoferrin in Capsules

Lactose (by Wako Pure Chemicals) (600 g), 400 g of corn starch (by Nisshin Flour Milling), 400 g of crystalline cellulose (by Wako Pure Chemicals) and 600 g of bovine lactoferrin (by Milei) were sieved using a 50-mesh sieve (by Yamato Kagaku), placed in a polyethylene bag having a thickness of 0.5 mm, mixed with tumbling and filled in capsules (by Nippon Elanco; gelatin capsule #1; Op. Yellow No.6 Body; empty weight: 75 mg) in an amount of 275 mg in each capsule to prepare 7,000 capsules each containing 82 mg of lactoferrin using an automatic capsule filling machine (by Cesere Pedini; Press type).

### Example 4: Preparation of Diluted Human Lactoferrin Powder

Lactose (by Morinaga Milk Industry) (50 g) and 10 g of human lactoferrin powder (by the same method as in Referential Example 1) previously sieved by a sieve #6 (by Inouchi Seieido) were mixed in a mortar, 40 g of lactose (by Morinaga Milk Industry) previously sieved by a sieve #5 (by Inouchi Seieido) were added thereto and mixed therewith, the whole mixture was sieved again by a sieve #5 and packed by a packing machine (OMP-90A: by Tokyo Shokai) so that each pack contained 5 g whereupon 20 packs of a 10% human lactoferrin powder were prepared.

### Example 5: Preparation of Tablets Compounded with Hydrolyzed Bovine Lactoferrin

| | |
|---|---|
| Hydrolysate of bovine lactoferrin (manufactured by the same method as in Referential Example 2) | 16.5 (%) |
| Lactose (by Morinaga Milk Industry) | 18.5 |
| Potato starch (by Kanto Kagaku) | 54.2 |
| Magnesium stearate (by NOF) | 1.4 |
| Carboxymethyl cellulose calcium (by Gotoku Yakuhin) | 9.4 |

A mixture of bovine lactoferrin, lactose, potato starch and carboxymethyl cellulose calcium was uniformly kneaded together with addition of sterilized water appropriately and dried at 55°C for 2 hours, then magnesium stearate was added to the resulting dry substance and mixed therewith and the mixture was tabletted by a common method to give tablets.

### Example 6: Preparation of Syrup Compounded with Hydrolyzed Bovine Lactoferrin

| | |
|---|---|
| Hydrolysate of lactoferrin (manufactured by the same method as in Referential Example 2) | 6.5(%) |
| Fructose/glucose liquid (by Sanmatsu Kogyo) | 14.0 |
| Citric acid (by Tanabe Seiyaku | 0.2 |
| Sodium citrate (by Iwaki Seiyaku) | 0.2 |
| Carboxymethyl cellulose calcium (by Gotoku Yakuhin) | 0.2 |
| Pure water (by Fuso Yakuhin Kogyo) | 78.9 |

The components were mixed and syrup was prepared by a common method.

### Industrial Applicability

The improving agent for hepatic functions according to the present invention is useful as a pharmaceutical agent for improvement of hepatic functions of patients having disorder of hepatic functions. In addition, when the improving agent for hepatic functions according to the present invention is used together with other therapeutic agents for hepatic diseases such as Interferon and SNMC, the agent of the present invention is useful for reducing the dose of such other therapeutic agents.

## Claims

1. An improving agent for hepatic functions containing a lactoferrin and/or a hydrolysate of lactoferrin as effective ingredient.

2. The improving agent for hepatic functions according to claim 1, which is lactoferrin selected from a group consisting of metal-unsaturated lactoferrin, metal-saturated lactoferrin and apolactoferrin or is a mixture of two or more thereof.
